# FASCICULE DE BREVET EUROPEEN

(11) **EP 1 244 427 B1**
(45) Date de publication et mention de la délivrance du brevet: **15.10.2003**
(21) Numéro de dépôt: 00993686.5
(22) Date de dépôt: 27.12.2000
(51) Int. Cl.: A61K 9/127

(54) **COMPOSITIONS PHARMACEUTIQUES DESTINEES A UNE ADMINISTRATION PAR VOIE ORALE**
PHARMAZEUTISCHE ZUSAMMENSETZUNGEN ZUR ORALEN VERABREICHUNG
PHARMACEUTICAL COMPOSITIONS FOR ORAL ADMINISTRATION

(30) Priorité: 03.01.2000 FR 0000015
(43) Date de publication de la demande: 02.10.2002
(73) Titulaire: CAPSULIS, 33600 Pessac (FR)
(72) Inventeur: GUINEZ, Charles, F-33000 Bordeaux (FR); LAVERSANNE, René, F-33600 Pessac (FR); AMEDEE, Jo[lle, F-33600 Pessac (FR)
(74) Mandataire: Giraud, Françoise
(86) Numéro de dépôt international: FR0003692
(87) Numéro de publication internationale: WO01049264

(56) Documents cités:
- WO-A-93/19737

## Description

La présente invention concerne de nouvelles compositions pharmaceutiques destinées à une administration par voie orale.

Aujourd'hui de nombreux médicaments nécessitent une administration parentérale, soit par injection intramusculaire, sous-cutanée ou intraveineuse, soit par incorporation dans une perfusion. En général une telle voie d'administration est rendue nécessaire par :
- Une fragilité de la molécule active vis à vis des conditions physico-chimiques et biologiques du tractus gastro-intestinal (pH, enzymes, ...),
- Une non perméabilité de la barrière gastro-intestinale à la molécule active,
- Une toxicité ou un trop fort pouvoir d'irritation de la molécule active vis à vis des muqueuses gastro-intestinales.

Parmi les molécules entrant dans la catégorie des molécules à administration parentérale obligatoire, on peut citer, par exemple :
- Les peptides et polypeptides, en particulier les hormones, qui sont décomposées par les milieux gastro-intestinaux,
- L'héparine qui ne passe pas la barrière,
- De nombreux cytostatiques connus pour leur toxicité et la non perméabilité de la barrière gastro-intestinale à leur égard.

D'autre part, d'autres tissus muqueux, qui pourraient être des sites privilégiés d'administration de médicaments ne sont pas utilisés comme tels, du fait de leur faible perméabilité aux molécules actives envisagées. Cela oblige parfois à utiliser soit une administration systémique, soit un fort surdosage du principe actif. Dans les deux cas, la dose administrée est nettement supérieure à la dose nécessaire, ce qui entraîne de nombreux inconvénients, en matière de coût, comme en matière de sécurité du médicament.

De plus, l'acceptation du traitement par le patient ainsi que le confort de ce dernier sont des paramètres de plus en plus pris en compte dans le développement de nouveaux médicaments. A ce titre, une administration non invasive, par voie orale, représente un progrès net dans ce sens.

Plusieurs approches ont été développées pour faciliter l'administration par voie orale des médicaments.

La plus classique, destinée à protéger le principe actif de l'acidité et de l'action enzymatique rencontrées dans l'estomac consiste à enrober la molécule active dans un comprimé dont la pellicule extérieure est résistante à un tel milieu. En général ledit enrobage se dissout une fois mis en conditions de pH neutre comme celles rencontrées dans l'intestin, permettant ainsi la libération de la matière active. Cette approche est efficace pour des molécules soit agissant directement dans l'intestin, soit ne présentant pas de difficultés au passage dans la circulation générale au travers de la muqueuse intestinale. Elle n'est donc pas efficace pour favoriser le transport trans-membranaire nécessaire à certaines molécules comme l'héparine. Elle ne s'applique pas non plus à des molécules sensibles aux enzymes présentes dans l'intestin (protéases en particulier qui détruisent les protéines).

Dérivées de cette technologie, les microcapsules enrobées de polymères permettent, outre la protection du principe actif, sa libération contrôlée ou retardée dans l'intestin, et donc une meilleure pharmacocinétique permettant par exemple de réduire les effets secondaires du produit. Le brevet européen EP 0 709 087 au nom de Flamel décrit une telle invention. Le diamètre des particules (de l'ordre de quelques centaines à quelques milliers de micromètres) peut être modifié, ainsi que l'épaisseur de l'enrobage, permettant des variations dans les profils cinétiques de libération.

De nombreux essais ont mis en oeuvre différentes technologies basées sur des microvésicules ou sur des microsphères ou des nanosphères de polymères afin de protéger le principe actif mais aussi de faciliter son passage au travers de la membrane gastro-intestinale, vers la circulation. On peut citer les liposomes (cf. M. Ueno *et al*., Chem. Pharm. Bull., **30** p. 2245-2247, 1982) utilisés pour faciliter le passage de l'héparine, ou les nanosphères de poly(alkyl cyanoacrylates) utilisées pour l'administration orale d'insuline (C. Damgé *et al*., J Pharma. Sc., **86** p. 1403-1409, 1997). Basés sur une autre technologie, des adjuvants comme les dérivés acétylés d'aminoacides (A. Leone-Bay *et al*., J. Controlled release, **50** p. 41-49, 1998) ou des hydrogels (J. M. Dunn & A. S. Hollister, Current Therap. Res. **56** p. 738-745, 1995) ont été utilisés pour faciliter l'administration orale d'héparine

Parmi les vésicules, objets sphériques formés d'arrangements moléculaires de molécules amphiphiles, les vésicules multilamellaires ont fait l'objet d'importantes recherches et ont donné lieu à plusieurs applications. A titre d'exemples, on citera les demandes internationales WO 93/19735 ; WO 95/18601 ; WO 97/00623 ; WO 98/02144 ; WO 99/16468. De telles vésicules désignées par vésicules multilamellaires à structure en oignon dans les documents cités ci-dessus se distinguent des liposomes par :
- leur mode de préparation qui part d'une phase lamellaire cristal-liquide, c'est à dire d'une phase à l'équilibre thermodynamique, présentant un ordre à longue distance,
- leur structure interne cristal-liquide lamellaire. Cette structure est formée d'un empilement de bicouches concentriques d'amphiphiles alternant avec des couches d'eau ou de solution aqueuse ou de solution d'un liquide polaire (glycérol par exemple), du centre jusqu'à la périphérie de ces vésicules. Avant leur dispersion dans un milieu d'utilisation, ces vésicules se trouvent dans un état d'équilibre thermodynamique. La structure spécifique de ces vésicules peut être aisément mise en évidence par microscopie optique, en particulier par microscopie optique en lumière polarisée,
- la nature variée des molécules amphiphiles qui peuvent être utilisées, seules ou en mélange, pour les constituer,
- les milieux dans lesquels on peut les disperser, qui peuvent être aussi bien hydrophiles que lipophiles, en fonction de la formulation.

La taille de ces vésicules multilamellaires est voisine du micromètre, typiquement de 0,1 µm à 20 µm en diamètre. Leur utilisation pour l'administration d'un médicament par voie orale n'a jamais été illustrée ni démontrée.

Les inventeurs de la présente invention ont maintenant découvert que l'administration par voie orale des vésicules multilamellaires dont la structure spécifique est définie ci-dessus, incorporant une molécule connue pour ne pas pouvoir être administrée par cette voie, soit du fait de sa fragilité, soit du fait de son incapacité à franchir la barrière gastro-intestinale était rendue possible lorsqu'elle était incorporée dans ces vésicules multilamellaires. Une telle incorporation favorise le passage de la molécule à travers la paroi gastro-intestinale et permet de la retrouver dans la circulation sanguine. Cette découverte a été faite à partir d'expériences menées sur des molécules de natures et caractéristiques très différentes, telles que l'héparine d'une part et la calcitonine, d'autre part.

Un avantage de l'invention est que ces vésicules multilamellaires sont préparées à partir de constituants biocompatibles connus pour leur innocuité.

Un autre avantage de l'invention est que le procédé de préparation des vésicules est de mise en oeuvre simple et ne fait appel qu'à des appareils courants de la chimie.

Par ailleurs, le fait que le procédé fasse appel à une phase lamellaire initiale à l'équilibre thermodynamique lui donne une excellente reproductibilité ainsi qu'une grande stabilité des vésicules obtenues.

Ainsi, selon l'une de ses caractéristiques essentielles, l'invention concerne l'utilisation de vésicules multilamellaires présentant une structure interne cristal-liquide lamellaire formée, du centre jusqu'à la périphérie desdites vésicules, d'un empilement de bicouches concentriques à base d'agents amphiphiles alternant avec des couches d'eau, de solution aqueuse ou de solution d'un liquide polaire et au sein desquelles se trouve incorporé au moins un principe actif, pour la fabrication d'une composition pharmaceutique destinée à une administration par voie orale.

Par le terme "incorporé" dont l'utilisation nous semble préférable à celle du terme "encapsulé", on entend que le ou les principes actifs font partie intégrante de l'entité constituée par la vésicule. En effet, on peut trouver des molécules de principe actif dans n'importe quelle couche comprise entre le centre et la périphérie de ladite vésicule.

L'expression "administration par voie orale" est utilisée dans son sens habituel et signifie que le produit est introduit dans l'organisme par la bouche, par opposition, par exemple, à un mode d'administration par voie parentérale.

Il est apparu, au cours des essais réalisés par les inventeurs, que l'incorporation d'un principe actif au sein des vésicules, présentait différents avantages déjà exposés dans ce qui précède. Les vésicules agissent, en particulier, comme de véritables vecteurs du principe actif, permettant de faciliter son passage à travers la barrière gastro-intestinale et/ou de le protéger de la dégradation, en particulier, de la dégradation par les enzymes présentes dans le milieu gastro-intestinal.

Les vésicules utilisées ont généralement des diamètres compris entre 0,1 et 25 µm, de préférence entre 0,2 et 15 µm.

Plus précisément, les vésicules utilisées selon l'invention sont de préférence constituées de plusieurs couches d'agents amphiphiles alternant avec des couches de phase aqueuse ou polaire. L'épaisseur de chacune de ces couches est une épaisseur moléculaire, typiquement de l'ordre de 5 à 10 nanomètres. Pour un empilement d'une dizaine à quelques centaines de couches, on obtient donc un diamètre compris entre 0,1 µm et quelques dizaines de micromètres. C'est ce qui est observé expérimentalement, les vésicules étant observables en microscopie optique (en lumière polarisée afin d'avoir un meilleur contraste lié à leur biréfringence), soit comme des points non résolus pour les plus petites d'entre elles, soit comme des sphères biréfringentes pour les plus grosses. Le profil de tailles peut être étudié à l'aide d'un granulomètre laser (utilisant la diffusion statique d'un faisceau laser, analysée sous plusieurs angles). On obtient en général un profil gaussien centré sur une valeur variant entre 0,1 et 25 µm montrant une faible hétérogénéité de la taille pour une formulation donnée, dans des conditions opératoires de préparation données.

Les vésicules dans lesquelles se trouve incorporé l'agent actif ont, comme exposé précédemment, une structure multilamellaire dite en oignon et sont constituées, de leur centre jusqu'à leur périphérie, d'une succession de couches lamellaires séparées par un milieu liquide. Ces vésicules peuvent être obtenues par un procédé comprenant la préparation d'une phase lamellaire cristal-liquide et sa transformation par application d'un cisaillement. Un tel procédé est en particulier décrit dans le brevet WO 93/19735 issu du brevet français FR 2 689 418 ou WO 95/18601 introduits ici par référence.

Selon le brevet français FR-2 689 418, cette transformation peut être faite lors d'une étape de cisaillement homogène de la phase cristal-liquide, ce qui conduit à des vésicules, encore appelées microcapsules, de taille contrôlée. Toutefois, en jouant sur la formulation de la phase lamellaire cristal-liquide, en particulier sur la nature des tensioactifs entrant dans sa composition, la transformation de cette phase cristal-liquide en vésicules peut être obtenue par simple sollicitation mécanique, en particulier lors du mélange des constituants.

De telles vésicules présentent, entre autres, l'avantage de pouvoir être préparées par un procédé de préparation particulièrement simple permettant d'utiliser une grande variété de tensioactifs.

Un autre avantage, lié lui aussi essentiellement au procédé utilisé pour préparer les vésicules dites à structure en oignon utilisées selon l'invention, réside dans le fait que l'on incorpore les actifs et les additifs préalablement à la formation des vésicules, ce qui permet un excellent rendement d'encapsulation, d'où une meilleure efficacité et une économie très importante pour des molécules coûteuses.

De telles structures sont avantageusement obtenues par incorporation d'au moins un agent actif dans une phase lamellaire cristal-liquide comprenant au moins un agent tensioactif puis transformation de cette phase cristal-liquide lamellaire en une phase dense de vésicules multilamellaires de petite taille.

Il est important de remarquer que cette transformation ne modifie pas la symétrie cristal-liquide de la phase initiale, et qui caractérise les vésicules multilamellaires. Cette symétrie cristal-liquide se traduit par des observations macroscopiques, telles que la biréfringence en microscopie optique, ou la présence de pic de diffraction en analyse par les rayons X. Il faut toutefois noter que de telles observations peuvent être rendues difficiles soit par le faible contraste lié à la très faible taille des vésicules, en microscopie optique, soit par la dilution et donc la faiblesse du signal en diffraction des rayons X.

Ainsi, les vésicules utilisées selon l'invention peuvent être obtenues selon un procédé selon lequel on prépare une phase cristal-liquide lamellaire incorporant au moins un agent actif et on provoque le réarrangement de ladite phase cristal-liquide en vésicules multilamellaires par application d'un cisaillement ou d'une sollicitation mécanique, résultant par exemple du mélange des constituants de ladite phase cristal-liquide lamellaire.

Ce cisaillement pourra être un cisaillement homogène, ce qui présente l'avantage de conduire à des vésicules de taille parfaitement homogène. Toutefois, une simple agitation mécanique pourra s'avérer suffisante pour conduire à la formation des vésicules multilamellaires de l'invention.

Les molécules amphiphiles utilisées pour la préparation des vésicules seront choisies, sans que cela soit une obligation, parmi les molécules faisant l'objet d'une description dans la pharmacopée, ou déjà utilisées dans des médicaments destinés à une administration par voie orale. On peut citer, sans que cela soit exhaustif, ni que les produits aient été obligatoirement utilisés précédemment en pharmacie, le groupe constitué :
◆ des glycérolipides, en particulier phospholipidiques, hydrogénés ou non
◆ des acides gras en C₆ à C₃₀, saturés ou mono- ou polyinsaturés, linéaires ou ramifiés, éthoxylés ou non, sous forme d'acide ou de sel d'un métal alcalin, alcalino-terreux ou d'une amine,
◆ des esters, éthoxylés ou non, de saccharose, ou de sorbitol, ou de mannitol, ou de glycérol ou de polyglycérol contenant de 2 à 20 motifs glycérol ou de glycol des acides gras ci-dessus,
◆ des mono-, di- ou triglycérides ou des mélanges de glycérides des acides gras ci-dessus,
◆ des alcools gras en C₆ à C₃₀, saturés ou mono- ou polyinsaturés, linéaires ou ramifiés, éthoxylés ou non,
◆ du cholestérol et ses dérivés, en particulier les esters de cholestérol chargés ou neutres comme le sulfate de cholestérol,
◆ des autres dérivés à squelette stérol, en particulier ceux d'origine végétale tels que le sitostérol ou le sigmastérol,
◆ des éthers, éthoxylés ou non, de saccharose, ou de sorbitol, ou de mannitol, ou de glycérol ou de polyglycérol contenant de 2 à 20 motifs glycérol ou de glycol et des alcools gras ci-dessus,
◆ des huiles végétales polyéthoxylées, hydrogénées ou non hydrogénées,
◆ des polymères séquencés de polyoxyéthylène et de polyoxypropylène (poloxamères),
◆ de l'hydroxystéarate de polyéthylèneglycol,
◆ des sphingolipides, et des dérivés de la sphingosine,
◆ des polyalkylglucosides,
◆ des céramides,
◆ des copolymères de polyéthylèneglycol et d'alkylglycol, par exemple, les copolymères de la famille des ELFACOS de AKZO NOBEL,
◆ des copolymères di- ou tribloc d'éthers de polyéthylèneglycol et de polyalkylèneglycol, par exemple les copolymères de la famille des ARLACELL de ICI.

Ces amphiphiles ou tensioactifs peuvent être utilisés seuls ou en mélange. Parmi ces amphiphiles, certains peuvent à eux seuls former une phase cristal-liquide lamellaire par mélange avec un solvant polaire. D'autres sont utilisés uniquement en mélange, en plus faible proportion, pour apporter des propriétés de rigidité ou d'étanchéité à la phase cristal-liquide lamellaire.

La formulation fait avantageusement intervenir un mélange de molécules tensioactives. Il est généralement utilisé au moins deux tensioactifs différents ayant des balances hydrophile-lipophile différentes, ce qui permet de régler en continu les propriétés des bicouches et ainsi de contrôler l'apparition de l'instabilité qui gouverne la formation des vésicules multilamellaires.

Ainsi, on choisira avantageusement parmi les tensioactifs ci-dessus, deux tensioactifs présentant des propriétés relativement différentes, en particulier une balance hydrophile-lipophile (HLB) différente. Le premier tensioactif présentera avantageusement une balance hydrophile-lipophile comprise entre 1 et 6, de préférence entre 1 et 4, alors que le deuxième tensioactif aura une balance hydrophile-lipophile comprise entre 3 et 15, de préférence comprise entre 5 et 15.

Un autre avantage des vésicules utilisées selon l'invention est que l'on peut ajouter à la formulation des polymères naturels ou artificiels tels que les polysaccharides (alginates, chitosan, etc.) afin de renforcer la solidité de la vésicule, et de lui permettre de mieux résister aux contraintes physico-chimiques du milieu d'administration (pH, effet mécanique, pression osmotique...). Ces polymères peuvent être aussi bien incorporés dans la vésicule, que déposés autour sous la forme d'un enrobage. Dans ce cas, la vésicule ou la particule formée des vésicules enrobées dans la matrice polymère a un diamètre plus gros que les vésicules seules. Ces polymères peuvent éventuellement être réticulés pour renforcer encore leur solidité.

Les vésicules utilisées selon l'invention, ont avantageusement un diamètre compris entre 0,1 et 25 µm, de préférence entre 0,2 et 15 µm.

Dans les compositions utilisées selon l'invention, les vésicules sont avantageusement dispersées au sein d'un milieu pharmaceutiquement acceptable, généralement constitué d'eau ou d'un tampon, ce milieu étant identique ou différent de celui que l'on trouve entre les lamelles des vésicules.

Les vésicules selon l'invention peuvent aussi être dispersées dans un milieu hydrophobe, par exemple une huile, acceptable pour l'usage pharmaceutique, par exemple une huile végétale ou minérale, un ester d'acide gras synthétique, ou le squalane ou squalène. Ceci peut avoir l'avantage d'éviter ou de limiter la présence d'eau dans le cas d'un actif qui serait par exemple hydrolysable. Cette dispersion dans l'huile peut elle-même être émulsionnée dans un milieu aqueux, afin d'obtenir une formulation plus agréable à avaler. Du fait de la présence de la phase intermédiaire huileuse, le milieu continu extérieur, par exemple aqueux, est séparé du milieu interne des vésicules, assurant ainsi une protection supplémentaire à l'actif incorporé dans les vésicules.

Selon un autre de ses aspects, l'invention concerne également le procédé de préparation des vésicules et des compositions utilisées selon l'invention.

La méthode de préparation consiste dans un premier temps à préparer par mélange une phase lamellaire cristal-liquide incorporant les différents constituants. Celle-ci s'obtient par simple mélange des ingrédients, dans un ordre déterminé par l'expérimentateur d'après les miscibilités de chacun des constituants. Il peut être nécessaire de chauffer certains constituants pâteux ou solides afin de faciliter leur incorporation. Dans ce cas on additionne la molécule active de préférence en fin de mélange afin de lui éviter de subir une température trop élevée, si elle est sensible à la température. On peut aussi préparer un mélange de tous les constituants sauf de la molécule active ou sa solution aqueuse sous forme d'un mélange " stock " qu'on utilisera selon les besoins pour préparer la phase lamellaire. La solution aqueuse peut contenir différents constituants destinés à assurer sa compatibilité biologique et en particulier des mélanges tampons mais également plusieurs molécules actives synergiques. La phase lamellaire ainsi préparée est ensuite soumise à un cisaillement modéré (de 0 à 1000s⁻¹) pendant un temps limité (de 0 à 60 minutes).

Dans la plupart des cas, ce cisaillement est obtenu directement par l'action du dispositif ayant servi au mélange.

Dans une troisième étape, la phase lamellaire cisaillée est dispersée dans un milieu final, en général de l'eau ou un tampon, identique à/ou différent de celui ayant servi lors de la préparation de la phase lamellaire. Ce milieu de dispersion peut aussi être un milieu non aqueux, soit polaire (glycérol, polyéthylène glycol, alkylène glycol...), soit hydrophobe (huiles...). Cette dispersion se fait généralement à température ambiante (20-25°C) par addition lente du milieu sur la phase lamellaire sous agitation constante.

Un conservateur et éventuellement d'autres additifs destinés à compléter la formulation galénique peuvent être ajoutés au produit.

Les exemples qui suivent illustrent très clairement l'intérêt de l'invention en mettant en évidence le passage dans la circulation sanguine de l'héparine préalablement incorporée dans des vésicules selon l'invention (exemple 1) alors que ce produit, en simple solution, ne franchit pas la barrière gastro-intestinale et de la calcitonine également incorporée dans des vésicules selon l'invention alors que le même produit non incorporé dans des vésicules est instable dans le milieu gastro-intestinal (voir exemple 2).

Ces exemples sont illustrés par les figures 1 à 4 en ce qui concerne l'héparine, les figures 1 à 3 donnant les résultats d'expériences effectuées sur le rat et la figure 4, les résultats d'expériences effectuées sur le chien et par la figure 5 dans le cas de la calcitonine (expériences sur le rat).

Dans les exemples qui suivent, les proportions données pour les constituants des différentes formulations sont indiquées, sauf indication contraire, en pourcentage en poids.

### EXEMPLE 1 : VESICULES INCORPORANT DE L'HEPARINE

### 1.a Formulation et préparation de vésicules incorporant de l'héparine

L'héparine utilisée dans ces exemples est une héparine non fractionnée, extraite de muqueuses intestinales de porc (SIGMA H-9399, 188 unités USP/mg).

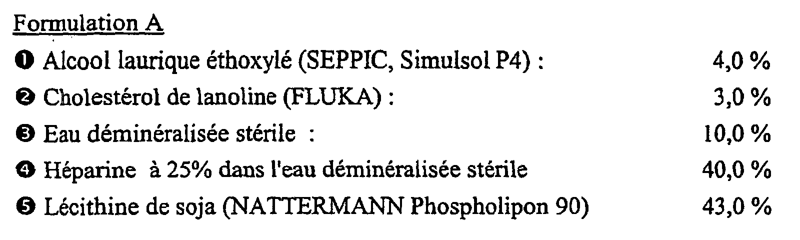

### Mode opératoire

Les constituants 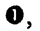 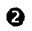 et 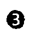 sont introduits dans un pilulier stérile de 2 ml, dans un ordre indifférent, puis mélangés à température ambiante à l'aide d'une spatule préalablement stérilisée à la flamme. La solubilisation totale du constituant 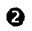 est vérifiée par observation microscopique. On introduit ensuite le constituant 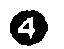 et on homogénéise à la spatule puis on ajoute le constituant 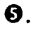

L'ensemble est homogénéisé à la spatule, puis laissé au repos une nuit à température ambiante.

La préparation est ensuite dispersée à 16,7 % dans de l'eau déminéralisée stérile. Le titre en héparine de la dispersion de vésicules est donc de 1,67% (16,7 mg/ml).

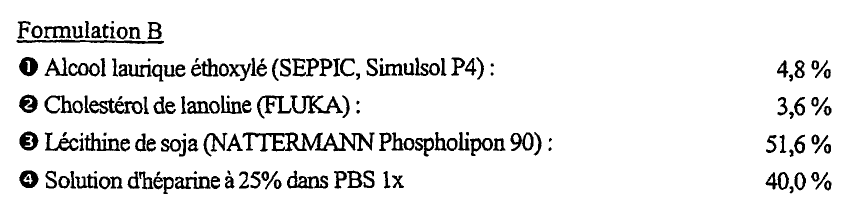

### Mode opératoire

Les constituants 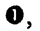 , 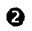 et 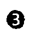 sont introduits dans un pilulier stérile de 2 ml, dans un ordre indifférent, puis solubilisés dans du dichlorométhane. Le solvant est ensuite évaporé totalement. On ajoute à température ambiante le constituant 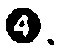 . L'ensemble est homogénéisé à l'aide d'une aiguille stérile, puis dispersé à 16,7 % dans du PBS 1x stérile. Le titre en héparine de la dispersion de vésicules est donc de 1,67% (16,7 mg/ml).

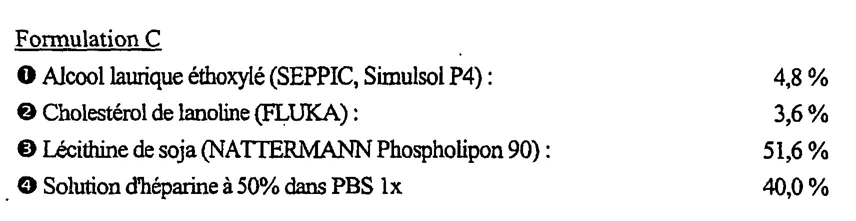

### Mode opératoire

Les constituants 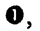 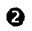 et 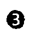 sont introduits dans un pilulier stérile de 2 ml, dans un ordre indifférent, puis solubilisés dans du dichlorométhane. Le solvant est ensuite évaporé totalement. On rajoute, à température ambiante, le constituant 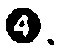 L'ensemble est homogénéisé à l'aide d'une aiguille stérile, laissé au repos une nuit à 4°C, puis dispersé à 16,7 % dans du PBS 1x stérile. Le titre en héparine de la dispersion de vésicules est donc de 3,34% (33,4 mg/ml).

### 1.b Caractérisation physico-chimique des vésicules incorporant de l'héparine

Les vésicules sont caractérisées d'une part par la mesure du profil granulométrique, et d'autre part par le rendement d'encapsulation.

### Profil de taille

Le profil de taille est étudié par granulométrie laser, en utilisant un appareil Mastersizer S de Malvern.

Pour l'échantillon A, le profil de taille montre un pic principal centré autour de 0,34 µm, et s'étendant de 50 nm à 20 µm avec un maximum secondaire autour de 6 µm. Environ 60% (en volume) de l'échantillon a une taille inférieure à 10 µm.

Pour l'échantillon B, le profil de taille montre un pic principal centré autour de 0,32 µm et s'étendant de 50 nm à 20 µm, avec un maximum secondaire autour de 3,5 µm. Environ 60% (en volume) de l'échantillon a une taille inférieure à 10 µm.

Pour l'échantillon C, le profil de taille montre un seul pic centré autour de 0,33 µm et s'étendant de 50 nm à 3 µm. Environ 85% (en volume) de l'échantillon a une taille inférieure à 1 µm.

L'analyse par diffusion laser est complétée par une observation en microscopie optique en lumière polarisée, qui permet de visualiser directement les vésicules. L'observation montre, pour les échantillons A et B, la présence d'une très grande majorité d'objets de taille voisine du micromètre et quelques objets plus gros. On observe aussi une tendance à l'agrégation des objets entre eux, sans aucune tendance à la coalescence.

### Rendement d'incorporation dans les vésicules

Le rendement d'incorporation est déterminé par séparation des vésicules de leur milieu de dispersion par centrifugation, puis analyse de la concentration en héparine dans le surnageant. Le titrage de l'héparine est effectué par un test colorimétrique (SIGMA Héparine Accucolor™ CRS106).

On obtient un rendement d'incorporation de 47 ± 5 % pour la formulation A, ce qui signifie qu'environ 50% de l'héparine introduite dans l'échantillon est à l'intérieur des vésicules de cette formulation A. Pour les formulations B et C, le rendement d'incorporation mesuré de la même manière est de 90 ± 5 %.

### 1.c Etude de l'administration chez le rat des vésicules incorporant de l'héparine

### Administration :

L'étude a été faite sur des rats Wistar

Les vésicules contenant de l'héparine et une solution témoin négatif sont administrées par voie orale, par gavage de l'animal non anesthésié en utilisant une aiguille à intubation.

Un témoin positif (solution aqueuse d'héparine) est injecté par voie sous-cutanée

Les animaux sont à jeun 24h avant le traitement (diète hydrique). L'administration a lieu à T_{0,} les prélèvements ayant lieu à T₀ (avant l'administration), puis à différents temps ultérieurs comme indiqué sur les figures annexées.

Les doses administrées et protocoles sont décrits dans le tableau suivant, pour chaque formulation :

| | Formulation A | Formulation B | Formulation C | |
|---|---|---|---|---|
| Rats | 5 rats mâles 400g | 2 rats mâles 400 g et 2 rats femelles 200 g | 2 rats mâles 400 g et 2 rats femelles 200 g | |
| Concentration de la dispersion | 16,7 mg/ml | 16,7 mg/ml | 33,4 mg/ml | |
| Dose administrée dans les vésicules | 2 ml/kg 33,4 mg/kg 6280 unités USP/kg | 1,2 ml/kg 20 mg/kg 3760 unités USP/kg | Dose I : 0,9 ml/kg 30 mg/kg 5640 unités USP/kg | Dose II : 3 ml/kg 100 mg/kg 18600 unités USP/kg |
| Solution témoin négatif | 16,7 mg/ml | | | |
| Dose témoin négatif voie orale | 2 ml/kg 33,3 mg/kg 6260 unités USP/kg | | | |
| Solution témoin positif voie SC | 2,4 mg/ml | 5 mg/ml | 5 mg/ml | |
| Dose témoin positif, voie SC | 1 ml/kg 2,4 mg/kg 450 unités USP/kg | 0,4 ml/kg 1,8 mg/kg 340 unités USP/kg | 0,4 ml/kg 1,8 mg/kg 340 unités USP/kg | |

### Prélèvement sanguin :

Les prélèvements sont réalisés par incision caudale (formulation A) ou par prélèvement rétro-orbital (formulations B et C). Un volume de 0,50 ml (formulation A) ou 1 ml (formulations B et C) de sang est recueilli sur tube au citrate de sodium à 3,8 %.

Le plasma est séparé du sang total par centrifugation à 92 g, 10 min à +4°C, juste après le prélèvement. Le plasma est conservé jusqu'à analyse par congélation à -18°C immédiatement après la séparation.

Le dosage quantitatif de l'héparine dans le plasma de rat ou de chien est effectué par une méthode colorimétrique (SIGMA Diagnostics® Héparine Accucolor™ Méthode n°CRS106). On réalise une courbe d'étalonnage afin de pouvoir exprimer en héparinémie les résultats obtenus en densité optique à 405 nm.

### Courbe d'étalonnage :

Pour la préparation des étalons d'héparine, on utilise un pool de plasmas prélevés sur des animaux à jeun, de la même manière que les échantillons à tester. On réalise quatre étalons d'héparine de concentration égale à 0, 0.226, 0.452 et 0.905 Unités/mL.

On reporte l'absorbance obtenue à 405 nm pour chaque étalon d'héparine en fonction de la concentration en héparine (Unités/mL). Le taux d'héparine d'un échantillon peut être déterminé à partir de cette courbe d'étalonnage.

### Résultats :

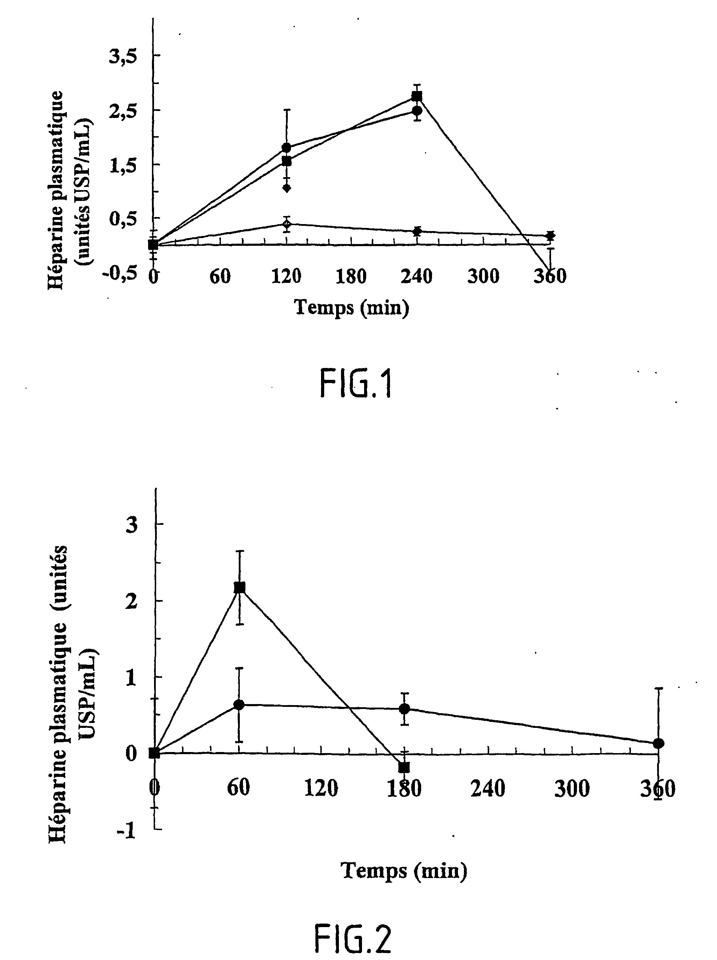

Les résultats sont donnés figures 1 à 3, où l'héparinémie (en Unités/mL), normalisée à 0 à T = 0 est portée en fonction du temps.

### Formulation A (figure 1)

On constate que la courbe obtenue par administration orale d'héparine incorporée dans les vésicules de l'invention augmente, pour atteindre un maximum et redescend ensuite. Ce phénomène est similaire à celui observé lors de l'injection sous-cutanée d'une solution d'héparine, montrée pour comparaison. Par contre l'administration par voie orale de la solution non encapsulée d'héparine ne produit aucun effet significatif sur l'héparinémie des animaux.

### Formulation B (figure 2)

On constate que la courbe obtenue par administration orale d'héparine incorporée dans les vésicules de l'invention augmente, pour atteindre un maximum environ 1 heure après la prise et redescend ensuite. Ce phénomène est similaire, mais plus marqué à celui observé lors de l'injection sous-cutanée de la solution d'héparine témoin positif, montrée pour comparaison.

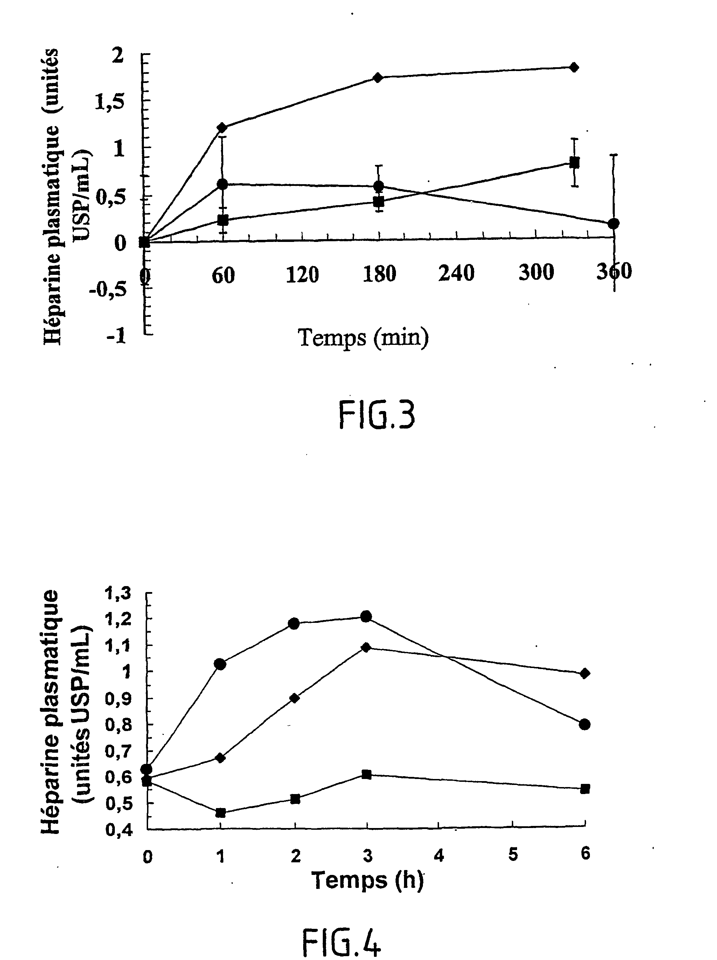

### Formulation C (figure 3)

On constate dans ce cas une croissance plus lente de la courbe obtenue par administration orale d'héparine incorporée dans les vésicules de l'invention, dont le maximum n'est pas encore atteint 6 heures après la prise. Ce temps d'activité long est remarquable et nettement supérieur à ce qui est observé dans le cas de l'injection sous-cutanée.

De plus, en comparant les deux courbes obtenues pour les doses I et II, on constate un effet « dose », l'héparinémie obtenue pour la dose II (18.600 unités USP/kg) étant nettement supérieure à celle correspondant à la dose I (5460 unités USP/kg).

### 1.d Etude de l'administration chez le chien des vésicules incorporant de l'héparine

Une étude analogue à celle de l'exemple précédent a été faite sur le chien, avec la formulation D ci-dessous :

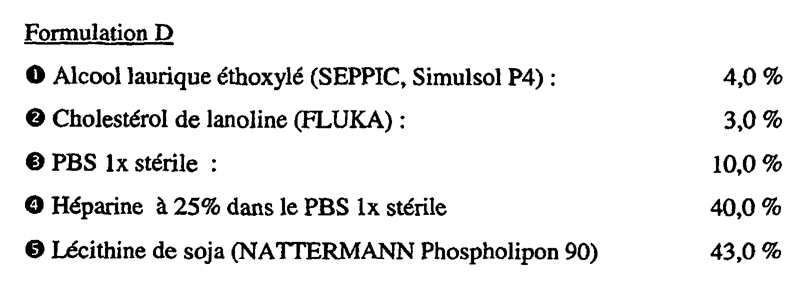

### Mode opératoire

Les constituants 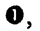 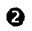 et 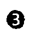 sont introduits dans un pilulier stérile de 2 ml, dans un ordre indifférent, puis mélangés par agitation magnétique pendant 2h. La solubilisation totale du constituant 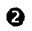 est vérifiée par observation microscopique. On introduit ensuite le constituant 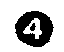 et on homogénéise à la spatule puis on ajoute le constituant 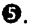

L'ensemble est homogénéisé à la spatule, puis laissé au repos une nuit à 4°C.

La préparation est ensuite dispersée à 10 % dans du PBS 1x stérile, ce qui conduit à un titre en héparine de la dispersion de 10 mg/ml.

### Protocole

Les chiens étaient des Beagle femelles, de 10 à 12 kg. Le protocole était le suivant :
- Volume administré par sonde oesophagienne de 3 ml/kg, soit pour les chiens utilisés un volume de 30 ml, correspondant à une dose en héparine dans les vésicules de 300 mg (30 mg/kg), i.e. 56 400 unités USP par chien (5 640 unités USP/kg),
- Les animaux sont à jeun 24h avant le traitement (diète hydrique). L'administration a lieu à T₀ les prélèvements réalisés au niveau de la veine jugulaire ayant lieu à T₀ (avant l'administration), T₀+1h, T₀+2h ou T₀+3h, T₀+6h.
- Témoin négatif : solution aqueuse d'héparine à 10 mg/ml ; volume administré de 30 ml, ce qui correspond à 300 mg d'héparine (30 mg/kg), soit 56 400 unités USP par chien (5 640 unités USP/kg),
- Témoin positif par injection sous-cutanée, solution à 10 mg/ml et volume d'injection de 0,2 ml/kg, soit pour les chiens utilisés 2 ml, ce qui correspond à une dose de 20 mg d'héparine (2 mg/kg), soit 3 800 unités USP par chien (380 unités USP/kg).
- L'étude du profil de taille des vésicules par granulométrie fait apparaître un maximum secondaire autour de 4,5 µm plus intense que dans le cas des. formulations A et B.

### Résultats :

Les résultats sont présentés, comme dans le cas du rat, sur la figure 4.

On observe, comme dans le cas du rat, que la courbe obtenue pour le chien ayant reçu l'héparine incorporée dans les vésicules par voie orale augmente dans des conditions similaires à celles observées avec la solution injectée par voie sous-cutanée. On remarque toutefois un léger décalage vers les temps longs de la courbe en fonction du temps lorsque l'héparine est incorporée dans des vésicules et administrée par voie orale. L'administration par voie orale d'une solution d'héparine n'a par contre aucune influence sur l'héparinémie.

### EXEMPLE 2 : VESICULES INCORPORANT DE LA CALCITONINE

### 2.a Préparation et caractérisation de vésicules incorporant de la calcitonine

Des expériences similaires ont été menées sur le rat, en incorporant de la calcitonine, qui est un peptide. Dans ce cas, la principale difficulté provient de la fragilité de la molécule, qui est très rapidement dégradée dans le milieu gastro-intestinal, dans le processus normal de digestion.

La calcitonine utilisée est une calcitonine de saumon (BACHEM H-2260), mise en solution aqueuse dans du PBS lx. La formulation et le mode opératoire de préparation des vésicules sont similaires à ceux utilisés dans le cas de l'héparine (formulation D).

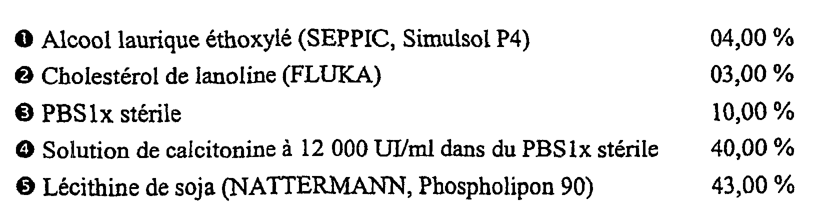

Les vésicules sont dispersées à 10% dans du PBS 1x stérile, ce qui correspond à un titre en calcitonine de 480 UI/ml.

Le profil granulométrique des vésicules montre une répartition de taille s'étendant de 50 nm à 10 µm, présentant un maximum principal centré sur 0,3 µm, et un maximum secondaire peu marqué autour de 3 µm. 85% de l'échantillon a une taille inférieure à 1 µm.

### 2.b Etude de l'administration chez le rat des vésicules incorporant la calcitonine.

L'étude a été réalisée sur des rats Wistar femelles de 150 g, répartis en groupes de 4 rats. L'administration par voie orale a été effectuée en utilisant une sonde oesophagienne, avec un volume d'administration de 300 µl, ce qui correspond à 144 UI administrée par animal.

Un groupe témoin positif a reçu par voie sous-cutanée un volume de 100 µl d'une solution aqueuse de calcitonine à 20 UI/ml, ce qui correspond à une dose de 2 UI de calcitonine par animal.

Un groupe témoin négatif a reçu par voie orale un volume de 300 µl d'une solution aqueuse de calcitonine titrée à 240 UI/ml, ce qui correspond à 72 UI par animal.

Les animaux ont été mis à jeun (diète hydrique) 24 heures avant l'expérience. Les prélèvements ont été effectués par voie rétro-orbitale, sous anesthésie générale à T₀ (avant l'administration), T₀+1h, T₀+2h ou T₀+3h, T₀+5h. 50 µl de sang sont prélevés dans des micro-capillaires héparinés.

La mesure du calcium et du pH sanguin est réalisée immédiatement après le prélèvement, directement sur le micro-capillaire de prélèvement, par un analyseur automatique CIBA-CORNING modèle 634, donnant la calcémie.

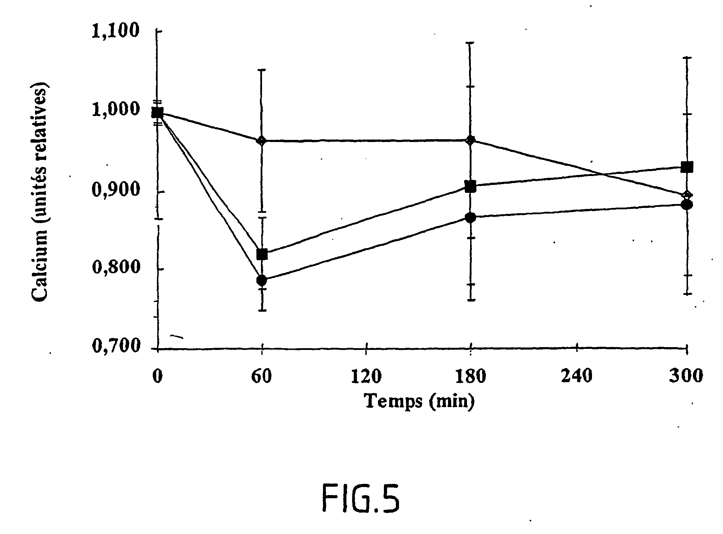

Les résultats sont résumés sur la figure 5. Dans le cas de la calcitonine incorporée dans les vésicules selon l'invention, administrée par voie orale, on observe une diminution de la calcémie au cours de la première heure, à environ 75% de sa valeur initiale, puis une remontée progressive dans l'heure qui suit. Ce comportement est similaire à celui obtenu avec l'injection sous-cutanée de la calcitonine en solution aqueuse. Par contre, ce phénomène n'est pas observé lors de l'administration par voie orale d'une solution de calcitonine non encapsulée. On peut donc conclure que l'encapsulation de la calcitonine selon l'invention a permis une protection de ce peptide vis à vis de la digestion en milieu gastro-intestinal ainsi qu'un passage à travers la muqueuse intestinale.

### LEGENDES DES FIGURES

FIGURE 1 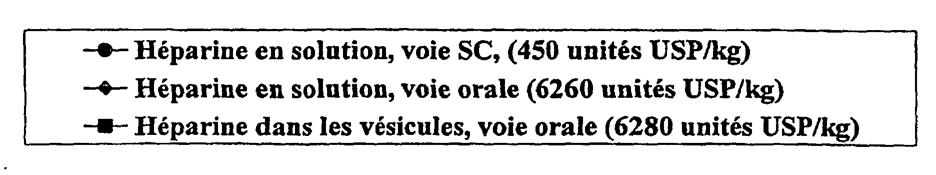
FIGURE 2 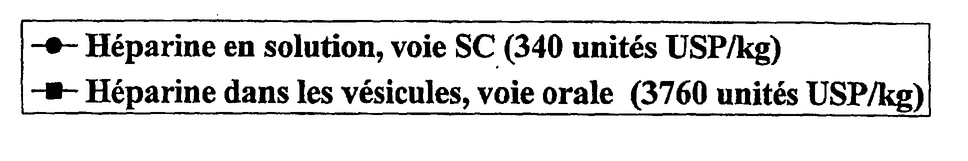
FIGURE 3 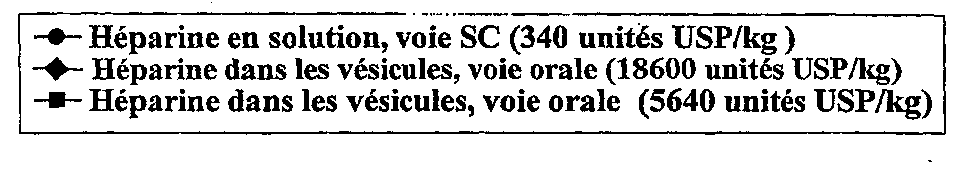
FIGURE 4 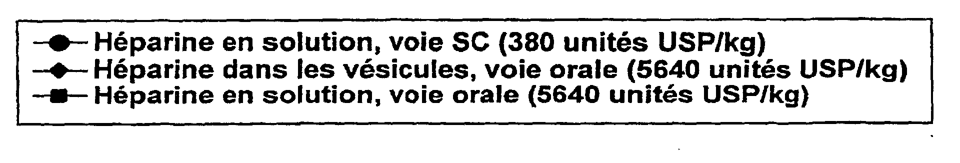
FIGURE 5

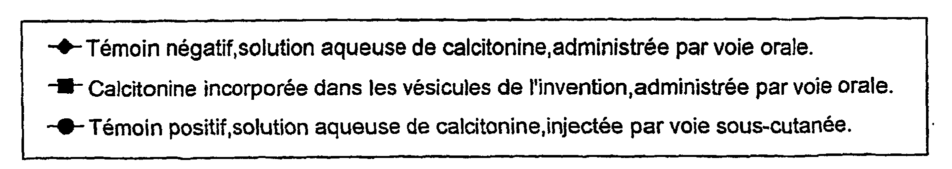

## Revendications

1. Utilisation de vésicules multilamellaires présentant une structure interne cristal-liquide lamellaire formée, du centre jusqu'à la périphérie desdites vésicules, d'un empilement de bicouches concentriques à base d'agents amphiphiles alternant avec des couches d'eau, de solution aqueuse ou de solution d'un liquide polaire et au sein desquelles se trouve incorporé au moins un principe actif, pour la fabrication d'une composition pharmaceutique destinée à une administration par voie orale.

2. Utilisation selon la revendication 1, **caractérisée en ce que** lesdites vésicules sont utilisées en tant que vecteurs dudit principe actif destinés à faciliter son passage à travers la barrière gastro-intestinale et/ou à le protéger de la dégradation, en particulier de la dégradation par les enzymes présentes dans le milieu gastro-intestinal.

3. Utilisation selon l'une des revendications 1 à 2, **caractérisée en ce que** ledit principe actif est l'héparine.

4. Utilisation selon l'une des revendications 1 ou 2, **caractérisée en ce que** ledit principe actif est un peptide.

5. Utilisation selon la revendication 4, **caractérisée en ce que** ledit peptide est une hormone peptidique.

6. Utilisation selon l'une des revendications 4 ou 5, **caractérisée en ce que** ledit principe actif est la calcitonine.

7. Utilisation selon l'une des revendications 1 à 6, **caractérisée en ce que** lesdites vésicules contiennent au moins un agent tensioactif choisi dans le groupe constitué :
◆ des glycérolipides, en particulier phospholipidiques, hydrogénés ou non
◆ des acides gras en C₆ à C₃₀, saturés ou mono- ou polyinsaturés, linéaires ou ramifiés, éthoxylés ou non, sous forme d'acide ou de sel d'un métal alcalin, alcalino-terreux ou d'une amine,
◆ des esters, éthoxylés ou non, de saccharose, ou de sorbitol, ou de mannitol, ou de glycérol ou de polyglycérol contenant de 2 à 20 motifs glycérol ou de glycol des acides gras ci-dessus,
◆ des mono-, di- ou triglycérides ou des mélanges de glycérides des acides gras ci-dessus,
◆ des alcools gras en C₆ à C₃₀, saturés ou mono- ou polyinsaturés, linéaires ou ramifiés, éthoxylés ou non,
◆ du cholestérol et ses dérivés, en particulier les esters de cholestérol chargés ou neutres comme le sulfate de cholestérol,
◆ des autres dérivés à squelette stérol, en particulier ceux d'origine végétale tels que le sitostérol ou le sigmastérol,
◆ des éthers, éthoxylés ou non, de saccharose, ou de sorbitol, ou de mannitol, ou de glycérol ou de polyglycérol contenant de 2 à 20 motifs glycérol ou de glycol et des alcools gras ci-dessus,
◆ des huiles végétales polyéthoxylées, hydrogénées ou non hydrogénées,
◆ des polymères séquencés de polyoxyéthylène et de polyoxypropylène (poloxamères),
◆ de l'hydroxystéarate de polyéthylèneglycol,
◆ des sphingolipides, et des dérivés de la sphingosine,
◆ des polyalkylglucosides,
◆ des céramides,
◆ des copolymères de polyéthylèneglycol et d'alkylglycol,
◆ des copolymères di- ou tribloc d'éthers de polyéthylèneglycol et de polyalkylèneglycol.

8. Utilisation selon l'une des revendications 1 à 7, **caractérisée en ce que** lesdites vésicules ont un diamètre compris entre 0,1 et 25 µm, de préférence entre 0,2 et 15 µm.

9. Utilisation selon l'une des revendications 1 à 8, **caractérisée en ce que** les bicouches desdites vésicules comprennent au moins deux agents tensioactifs dont l'un présente une balance hydrophile-lipophile (HLB) comprise entre 1 et 6, de préférence comprise entre 1 et 4, et l'autre une balance hydrophile-lipophile (HLB) comprise entre 3 et 15, de préférence comprise entre 5 et 15.

10. Utilisation selon l'une des revendications 1 à 9, **caractérisée en ce que** lesdites vésicules contiennent en outre un polymère incorporé dans lesdites vésicules ou déposé autour desdites vésicules sous forme d'un enrobage.

11. Utilisation selon l'une des revendications 1 à 10, **caractérisée en ce que** ladite composition comprend une dispersion desdites vésicules au sein d'un milieu pharmaceutiquement acceptable.

12. Utilisation selon la revendication 11, **caractérisée en ce que** ledit milieu pharmaceutiquement acceptable est constitué par de l'eau ou un tampon.

13. Utilisation selon la revendication 11, **caractérisée en ce que** ledit milieu pharmaceutiquement acceptable est constitué d'un milieu hydrophobe tel qu'une huile.

14. Utilisation selon la revendication 11, **caractérisée en ce que** ladite composition pharmaceutique comprend une émulsion dans un milieu aqueux desdites vésicules elles-mêmes préalablement dispersées dans une huile.

15. Utilisation selon l'une des revendications 1 à 14, **caractérisée en ce que** lesdites vésicules sont obtenues par transformation d'une phase cristal-liquide lamellaire incorporant ledit principe actif, sous l'action d'un cisaillement ou d'une sollicitation mécanique.

16. Composition pharmaceutique destinée à une administration orale et contenant de l'héparine à titre de principe actif, **caractérisée en ce que** ladite héparine se trouve incorporée au sein de vésicules multilamellaires telles que définies dans la revendication 1 ou dans l'une des revendications 7 à 10 ou telles qu'obtenues selon le procédé défini dans la revendication 15, lesdites vésicules étant dispersées dans un milieu pharmaceutiquement acceptable tel que défini dans l'une des revendications 11 à 14.

17. Composition pharmaceutique destinée à une administration orale et contenant de la calcitonine à titre de principe actif, **caractérisée en ce que** ladite calcitonine se trouve incorporée au sein de vésicules multilamellaires telles que définies dans la revendication 1 ou dans l'une des revendications 7 à 10 ou telles qu'obtenues selon le procédé défini dans la revendication 15, lesdites vésicules étant dispersées dans un milieu pharmaceutiquement acceptable tel que défini dans l'une des revendications 11 à 14.

## Patentansprüche

1. Verwendung von multilamellaren Vesikula, die eine innere Flüssigkristall-Lamellenstruktur aufweisen, die vom Zentrum bis zum Umfang der Vesikula besteht aus übereinander angeordneten konzentrischen Doppelschichten auf Basis von amphiphilen Agentien, die mit Schichten aus Wasser, einer wässrigen Lösung oder einer Lösung einer polaren Flüssigkeit abwechseln und in deren Innern mindestens ein Wirkstoff enthalten ist, für die Herstellung einer pharmazeutischen Zusammensetzung, die für eine Verabreichung auf oralem Wege bestimmt ist.

2. Verwendung nach Anspruch 1, **dadurch gekennzeichnet, dass** die Vesikula als Träger des Wirkstoffes verwendet werden, die dazu dienen sollen, dessen Durchgang durch die gastrointestinale Sperrschicht zu erleichtern und/oder ihn gegen Abbau, insbesondere gegen Abbau durch Enzyme, die in dem gastrointestinalen Medium enthalten sind, zu schützen.

3. Verwendung nach einem der Ansprüche 1 bis 2, **dadurch gekennzeichnet, dass** der Wirkstoff Heparin ist.

4. Verwendung nach einem der Ansprüche 1 oder 2, **dadurch gekennzeichnet, dass** der Wirkstoff ein Peptid ist.

5. Verwendung nach Anspruch 4, **dadurch gekennzeichnet, dass** das Peptid ein Peptidhormon ist.

6. Verwendung nach einem der Ansprüche 4 oder 5, **dadurch gekennzeichnet, dass** der Wirkstoff Calcitonin ist.

7. Verwendung nach einem der Ansprüche 1 bis 6, **dadurch gekennzeichnet, dass** die Vesikula mindestens ein Tensid enthalten, das ausgewählt ist aus der Gruppe, die besteht aus:
◆ hydrierten oder nicht-hydrierten Glycerolipiden, insbesondere Phospholipid-Glycerolipiden,
◆ gesättigten oder einfach oder mehrfach ungesättigten, linearen oder verzweigten, ethoxylierten oder nicht-ethoxylierten C₆-C₃₀-Fettsäuren in Form der Säure oder in Form eines Alkalimetall-, Erdalkalimetall- oder Aminsalzes,
◆ ethoxylierten oder nicht-ethoxylierten Saccharose- oder Sorbit- oder Mannit- oder Glycerin- oder Polyglycerin-Estem, die 2 bis 20 Glycerin-Einheiten enthalten, oder Glycol-Estern der oben genannten Fettsäuren,
◆ Mono-, Di- oder Triglyceriden oder Glycerid-Mischungen der oben genannten Fettsäuren,
◆ gesättigten oder einfach- oder mehrfach ungesättigten, linearen oder verzweigten, ethoxylierten oder nicht-ethoxylierten C₆-C₃₀-Fettalkoholen,
◆ Cholesterin und seinen Derivaten, insbesondere den geladenen oder neutralen Cholesterinestern wie Cholesterinsulfat,
◆ anderen Derivaten mit einem Sterin-Grundgerüst, insbesondere solche pflanzlichen Ursprungs wie Sitosterin oder Stigmasterin,
◆ ethoxylierten oder nicht-ethoxylierten Ethern von Saccharose oder Sorbit oder Mannit oder Gycerin oder Polyglycerin, enthaltend 2 bis 20 Glycerin-Einheiten, oder von Glycol und den oben genannten Fettalkoholen,
◆ polyethoxylierten, hydrierten oder nicht hydrierten Pflanzenölen,
◆ Sequenzpolymeren von Polyoxyethylen und Polyoxypropylen (Poloxameren),
◆ Polyethylenglycolhydroxystearat,
◆ Sphingolipiden und Sphingosin-Derivaten,
◆ Polyalkylglucosiden,
◆ Ceramiden,
◆ Copolymeren von Polyethylenglycol und Alkylglycol,
◆ Di- oder Triblock-Copolymeren von Polyethylenglycol- und Polyalkylenglycol-ethern.

8. Verwendung nach einem der Ansprüche 1 bis 7, **dadurch gekennzeichnet, dass** die Vesikula einen Durchmesser zwischen 0,1 und 25 µm, vorzugsweise zwischen 0,2 und 15 µm, haben.

9. Verwendung nach einem der Ansprüche 1 bis 8, **dadurch gekennzeichnet, dass** die Doppelschichten der Vesikula mindestens zwei Tenside enthalten, von denen eines ein hydrophiles-lipophiles Gleichgewicht (HLB-Wert) zwischen 1 und 6, vorzugsweise zwischen 1 und 4, aufweist und das andere ein hydrophiles-lipophiles Gleichgewicht (HLB-Wert) zwischen 3 und 15, vorzugsweise zwischen 5 und 15, aufweist.

10. Verwendung nach einem der Ansprüche 1 bis 9, **dadurch gekennzeichnet, dass** die Vesikula außerdem ein Polymer enthalten, das den Vesikula einverleibt worden ist oder um die Vesikula herum in Form einer Hülle abgeschieden worden ist.

11. Verwendung nach einem der Ansprüche 1 bis 10, **dadurch gekennzeichnet, dass** die Zusammensetzung eine Dispersion der Vesikula im Innern eines pharmazeutisch akzeptablen Mediums umfasst.

12. Verwendung nach Anspruch 11, **dadurch gekennzeichnet, dass** das pharmazeutisch akzeptable Medium aus Wasser oder einem Puffer besteht.

13. Verwendung nach Anspruch 11, **dadurch gekennzeichnet, dass** das pharmazeutisch akzeptable Medium aus einem hydrophoben Medium, beispielsweise einem Öl, besteht.

14. Verwendung nach Anspruch 11, **dadurch gekennzeichnet, dass** die pharmazeutische Zusammensetzung eine Emulsion der Vesikula, die vorher selbst in einem Öl dispergiert worden sind, in einem wässrigen Medium umfasst.

15. Verwendung nach einem der Ansprüche 1 bis 14, dass die Vesikula hergestellt worden sind durch Umwandlung aus einer lamellaren Flüssigkristallphase, die den Wirkstoff enthält, unter der Einwirkung einer Scherwirkung oder einer mechanischen Benspruchung.

16. Pharmazeutische Zusammensetzung, die bestimmt ist für eine orale Verabreichung und Heparin als Wirkstoff enthält, **dadurch gekennzeichnet, dass** das Heparin sich im Innern der multilamellaren Vesikula befindet, wie sie im Anspruch 1 oder in einem der Ansprüche 7 bis 10 definiert sind oder wie sie bei dem Verfahren nach Anspruch 15 erhalten werden, wobei die Vesikula in einem pharmazeutisch akzeptablen Medium, wie es in einem der Ansprüche 11 bis 14 definiert ist, dispergiert sind.

17. Pharmazeutische Zusammensetzung, die bestimmt ist für eine orale Verabreichung und Calcitonin als Wirkstoff enthält, **dadurch gekennzeichnet, dass** das Calcitonin im Innern der multilamellaren Vesikula enthalten ist, wie sie im Anspruch 1 oder in einem der Ansprüche 7 bis 10 definiert sind oder wie sie bei dem Verfahren nach Anspruch 15 erhalten werden, wobei die Vesikula in einem pharmazeutisch akzeptablen Medium dispergiert sind, wie es in einem der Ansprüche 11 bis 14 definiert ist.

## Claims

1. Use of multilamellar vesicles having a lamellar liquid-crystal internal structure formed, from the center to the periphery of said vesicles, of a stack of concentric bilayers based on amphiphilic agents alternating with layers of water, aqueous solution or solution of a polar liquid, with at least one active principle being incorporated inside said vesicles, for the manufacture of a pharmaceutical composition for oral administration.

2. Use according to claim 1, **characterized in that** said vesicles are used as vectors for said active principle, making it possible to facilitate its passage through the gastrointestinal barrier and/or to protect it from degradation, particularly degradation by the enzymes present in the gastrointestinal medium.

3. Use according to claim 1 or 2, **characterized in that** said active principle is heparin.

4. Use according to claim 1 or 2, **characterized in that** said active principle is a peptide.

5. Use according to claim 4, **characterized in that** said peptide is a peptide hormone.

6. Use according to claim 4 or 5, **characterized in that** said active principle is calcitonin.

7. Use according to one of claims 1 to 6, **characterized in that** said vesicles contain at least one surfactant selected from the group consisting of:
◆ hydrogenated or non-hydrogenated glycerolipids, particularly phospholipidic glycerolipids,
◆ ethoxylated or non-ethoxylated, linear or branched, saturated or mono- or polyunsaturated C₆ to C₃₀ fatty acids in the form of the acid or an alkali metal, alkaline earth metal or amine salt,
◆ ethoxylated or non-ethoxylated esters of sucrose, sorbitol, mannitol, glycerol or polyglycerol containing from 2 to 20 glycerol units, or glycol with the above fatty acids,
◆ mono-, di- or triglycerides or mixtures of glycerides of the above fatty acids,
◆ ethoxylated or non-ethoxylated, linear or branched, saturated or mono- or polyunsaturated C₆ to C₃₀ fatty alcohols,
◆ cholesterol and derivatives thereof, particularly charged or neutral cholesterol esters such as cholesterol sulfate,
◆ other derivatives with a sterol skeleton, particularly those of vegetable origin such as sitosterol or sigmasterol,
◆ ethoxylated or non-ethoxylated ethers of sucrose, sorbitol, mannitol, glycerol or polyglycerol containing from 2 to 20 glycerol units, or glycol with the above fatty alcohols,
◆ hydrogenated or non-hydrogenated, polyethoxylated vegetable oils,
◆ polyoxyethylene/polyoxypropylene block polymers (poloxamers),
◆ polyethylene glycol hydroxystearate,
◆ sphingolipids and sphingosine derivatives,
◆ polyalkyl glucosides,
◆ ceramides,
◆ polyethylene glycol/alkyl glycol copolymers,
◆ polyethylene glycol/polyalkylene glycol ether di-block or tri-block copolymers.

8. Use according to one of claims 1 to 7, **characterized in that** said vesicles have a diameter of between 0.1 and 25 µm, preferably of between 0.2 and 15 µm.

9. Use according to one of claims 1 to 8, **characterized in that** the bilayers of said vesicles comprise at least two surfactants, one of which has a hydrophilic-lipophilic balance (HLB) of between 1 and 6, preferably of between 1 and 4, while the other has a hydrophilic-lipophilic balance (HLB) of between 3 and 15, preferably of between 5 and 15.

10. Use according to one of claims 1 to 9, **characterized in that** said vesicles also contain a polymer incorporated in said vesicles or deposited around said vesicles in the form of a coating.

11. Use according to one of claims 1 to 10, **characterized in that** said composition comprises a dispersion of said vesicles in a pharmaceutically acceptable medium.

12. Use according to claim 11, **characterized in that** said pharmaceutically acceptable medium consists of water or a buffer.

13. Use according to claim 11, **characterized in that** said pharmaceutically acceptable medium consists of a hydrophobic medium such as an oil.

14. Use according to claim 11, **characterized in that** said pharmaceutical composition comprises an emulsion of said vesicles in an aqueous medium, the vesicles themselves having been dispersed in an oil beforehand.

15. Use according to one of claims 1 to 14, **characterized in that** said vesicles are obtained by transformation of a lamellar liquid-crystal phase incorporating said active principle under the action of shear or mechanical stress.

16. Pharmaceutical composition for oral administration containing heparin as the active principle, **characterized in that** said heparin is incorporated in multilamellar vesicles as defined in claim 1 or in one of claims 7 to 10, or as obtained by the process defined in claim 15, said vesicles being dispersed in a pharmaceutically acceptable medium as defined in one of claims 11 to 14.

17. Pharmaceutical composition for oral administration containing calcitonin as the active principle, **characterized in that** said calcitonin is incorporated in multilamellar vesicles as defined in claim 1 or in one of claims 7 to 10, or as obtained by the process defined in claim 15, said vesicles being dispersed in a pharmaceutically acceptable medium as defined in one of claims 11 to 14.
